# EUROPEAN PATENT APPLICATION

(11) **EP 4 825 960 A1**
(43) Date of publication of application: **21.10.2026**
(21) Application number: 24903720.1
(22) Date of filing: 11.12.2024
(51) Int. Cl.: C12N 5/10, C07K 16/00, C12N 15/13, C12P 21/08

(54) **FACTOR CAPABLE OF AFFECTING PRODUCTION OF SUBSTANCE USING CHO CELL**

(30) Priority: 14.12.2023 JP 2023211379
(71) Applicant: AGC Inc., Chiyoda-ku Tokyo 100-8405 (JP)
(72) Inventor: SHIBAFUJI, Yusuke, Tokyo 100-8405 (JP); NAGAO, Nobuyoshi, Tokyo 100-8405 (JP); DATE, Risa, Tokyo 100-8405 (JP)
(74) Representative: TBK
(86) International application number: PCT/JP2024/043842
(87) International publication number: WO 2025/127066

(57) **Abstract**

The present invention provides mammalian cells that produce a target protein, characterized in that the expression of proteins related to endoplasmic reticulum stress and/or proteins related to transcription is regulated in the cells.

## Description

### [Technical Field]

The present invention relates to a mammalian cell with improved production capacity of a target protein.

### [Background Art]

In the field of life sciences, the technology to produce useful proteins on an industrial scale by mass-culturing cells capable of producing the target useful proteins is very important. Examples of useful proteins include protein-based pharmaceutical products such as physiologically active substances such as insulin and erythropoietin, antibodies and the like.

When the aforementioned physiologically active substances are used as protein-based pharmaceutical products, relatively small doses for patients are sufficient to achieve effects. On the other hand, antibody drugs require doses in several hundred milligrams per administration. Therefore, there remains a high demand for the development of technologies to efficiently produce antibody drugs.

Various cell lines have been used in the production of antibody drugs, and the most frequently used one today is CHO (Chinese hamster ovary) cell. CHO cells have also been used for biopharmaceutical products other than antibodies, and are said to be the most compatible production cells for antibody drugs from the viewpoints of high productivity, quality of glycan structure, and safety (Non Patent Literature 1). A plurality of techniques for improving the antibody production capacity of CHO cells have been reported to date (Non Patent Literatures 2, 3), and many modified strains have been created. However, there still remains a high demand for the creation of a more preferred modified strain.

### [Citation List]

### [Non Patent Literature]

[Non Patent Literature 1]
   Kunert R et al., Appl Microbiol Biotechnol. 2016 Apr; 100(8):3451-61.
[Non Patent Literature 2]
   Porter AJ et al., Biotechnol Prog. 2010 Sep-Oct; 26(5):1446-54.
[Non Patent Literature 3]
   Feary M et al., Biotechnol Prog. 2021 Jul; 37(4):e3137.

### [Summary of Invention]

### [Technical Problem]

Generally, cell lines capable of producing a target protein are constructed using, as host cells, cells into which the target gene has been stably introduced. However, since plasmids containing the target gene are randomly inserted into the chromosomes of the host cells after transfection, the expression level of the target protein is known to vary greatly among production clones. Therefore, the process of selecting clones with high expression levels requires a vast amount of time and effort. While various factors determine the expression level of the target protein in a cell, it is assumed that the efficiency of each process influences the expression level, such as the copy number of the gene inserted into the chromosome, the transcription process to mRNA, the translation process to polypeptides, protein modification/folding, secretion outside the cell, and the like. However, the characteristics of cells that produce high levels of protein are not yet sufficiently understood. Therefore, the present inventors focused on low-producer clones in the cell pool after transfection with a plasmid containing the target gene, and compared the results of proteomics analysis of these clones with those of high-producer clones, thereby to elucidate the characteristics of cells capable of high protein production at both the gene level and the cell level, and as a result, to construct a means for easily selecting or producing cells that can efficiently produce the target protein.

### [Solution to Problem]

The present inventors have conducted intensive studies of the above-mentioned problems and found that the expression levels of specific proteins related to endoplasmic reticulum stress (PERK, Erp44, Txndc12, Pdia5, Pedx4, Edem3, Erlec1, and P3h4) were increased in low-productivity clones compared to high-productivity clones. In addition, they have found that the expression level of a specific protein related to transcription (TAF8) was decreased in low-productivity clones. Based on such findings, the present inventors have conducted further studies and completed the present invention.

That is, the present invention provides the following.

[1] A mammalian cell that produces a target protein, wherein the expression of a protein related to endoplasmic reticulum stress is regulated in the aforementioned cell.
[2] The cell of [1], wherein the aforementioned mammalian cell is a Chinese hamster ovary (CHO) cell.
[3] The cell of [1] or [2], wherein the aforementioned target protein is an antibody.
[4] The cell of any of [1] to [3], wherein the expression of the aforementioned protein related to the endoplasmic reticulum stress is suppressed.
[5] The cell of any of [1] to [4], wherein the aforementioned protein related to the endoplasmic reticulum stress is an endoplasmic reticulum stress sensor protein.
[6] The cell of [5], wherein the endoplasmic reticulum stress sensor protein is PERK.
[7] The cell of any of [1] to [4], wherein the aforementioned protein related to the endoplasmic reticulum stress is a protein that has the function of promoting the formation of a disulfide bond of the target protein.
[8] The cell of [7], wherein the aforementioned protein that has the function of promoting the formation of a disulfide bond of the target protein is at least one selected from the group consisting of Erp44, Txndc12, and Pdia5.
[9] The cell of any of [1] to [4], wherein the aforementioned protein related to the endoplasmic reticulum stress is a protein that has the function of reducing oxidative stress.
[10] The cell of [9], wherein the aforementioned protein that has the function of reducing oxidative stress is Prdx4.
[11] The cell of any of [1] to [4], wherein the aforementioned protein related to the endoplasmic reticulum stress is a protein that has the function of degrading a denatured protein.
[12] The cell of [11], wherein the aforementioned protein that has the function of degrading a denatured protein is at least one selected from the group consisting of Edem3 and Erlec1.
[13] The cell of any of [1] to [4], wherein the aforementioned protein related to the endoplasmic reticulum stress is a protein related to endoplasmic reticulum-phagy.
[14] The cell of [13], wherein the aforementioned protein related to the endoplasmic reticulum-phagy is P3h4.
[15] A mammalian cell that produces a target protein, wherein the expression of a protein related to transcription is regulated in the aforementioned cell.
[16] The cell of [15], wherein the aforementioned mammalian cell is a Chinese hamster ovary (CHO) cell.
[17] The cell of [15] or [16], wherein the aforementioned target protein is an antibody.
[18] The cell of any of [15] to [17], wherein the expression of the aforementioned protein related to the transcription is enhanced.
[19] The cell of any of [15] to [18], wherein the aforementioned protein related to the transcription is TAF8.
[20] A method for producing a target protein, comprising a step of culturing the cell of any of [1] to [19].
[21] A target protein produced by the cell of any of [1] to [19].

### [Advantageous Effects of Invention]

According to the present invention, a cell capable of efficiently producing a target protein can be easily selected or produced.

### [Brief Description of Drawings]

[Fig. 1]

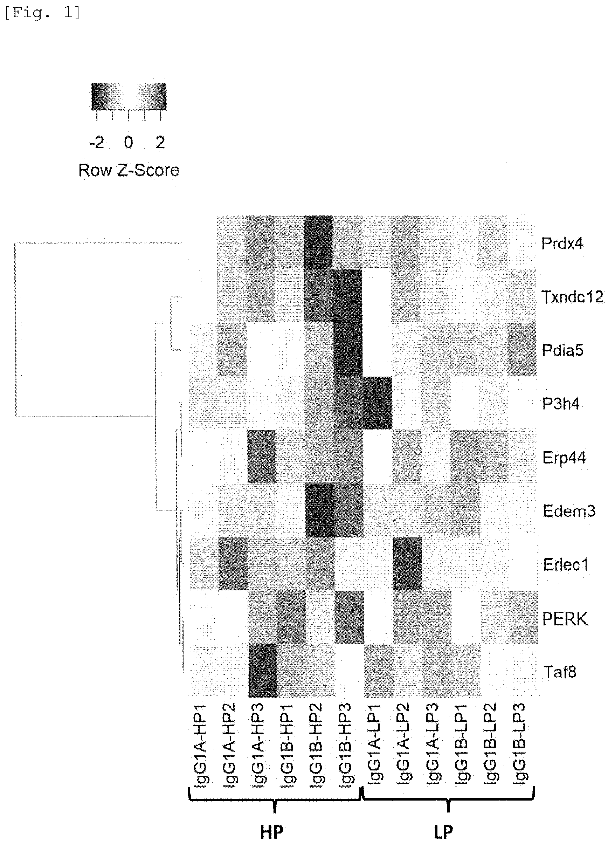

   Fig. 1 shows the proteins whose expression levels differed between high-producer (HP) clones and low-producer (LP) clones. Compared to standard conditions, red indicates protein upregulation and blue indicates protein downregulation. The left half of the Figure shows high-producer clones (HP), and the right half shows low-producer clones (LP).
[Fig. 2]

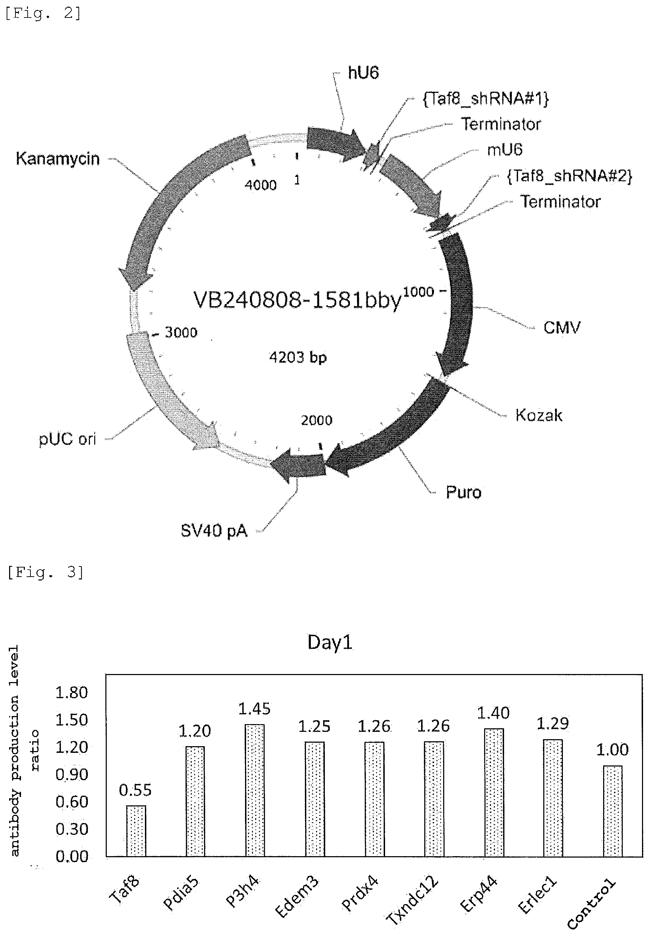

   Fig. 2 shows the vector map of the shRNA expression vector targeting the TAF8 gene, which was produced in Example 2.
[Fig. 3]
   Fig. 3 shows changes in the antibody production level by CHO cells (Day 1) when each target gene was knocked down.
[Fig. 4]

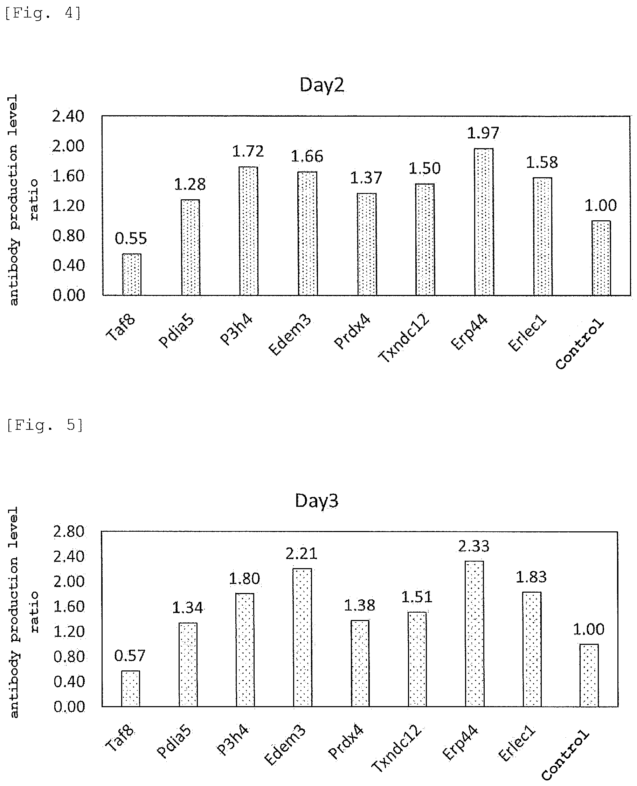

   Fig. 4 shows changes in the antibody production level by CHO cells (Day 2) when each target gene was knocked down.
[Fig. 5]
   Fig. 5 shows changes in the antibody production level by CHO cells (Day 3) when each target gene was knocked down.
[Fig. 6]

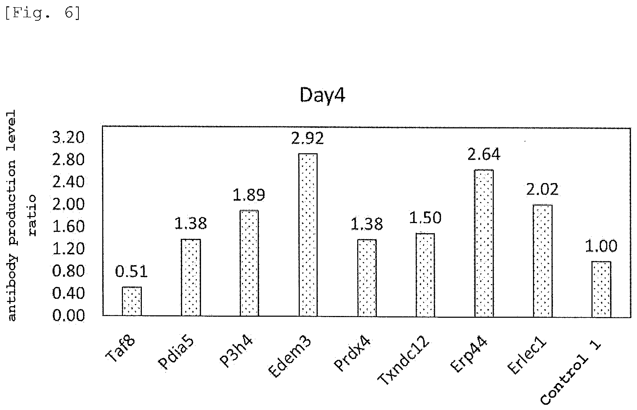

   Fig. 6 shows changes in the antibody production level by CHO cells (Day 4) when each target gene was knocked down.

### [Description of Embodiments]

The present invention is described in detail below.

### 1. Mammalian cell 1 that produces target protein

The present invention provides a mammalian cell that produces a target protein, wherein the expression of a protein related to endoplasmic reticulum stress is regulated in the aforementioned cell (hereinafter sometimes referred to as "the mammalian cell 1 of the present invention").

The mammalian cell 1 of the present invention is characterized in that the expression of a protein related to endoplasmic reticulum stress is regulated. In the present specification, "protein expression is regulated" means "protein expression is increased" or "protein expression is decreased". In one embodiment, "protein expression is regulated" means "during the production of a target protein, expression of at least one protein related to endoplasmic reticulum stress is increased by 1.1-fold or more (e.g., 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 3.0-fold or more) compared to the expression thereof in the cell before regulation", or "during the production of a target protein, expression of at least one protein related to endoplasmic reticulum stress is decreased by 10% or more (e.g., 20% or more, 30% or more, 40% or more, 50% or more, 60% or more, 70% or more, 80% or more, 90% or more) compared to the expression thereof in the cell before regulation". In addition, "protein expression is regulated" can be interpreted as "protein activity is regulated".

In one preferred embodiment of the present invention, the mammalian cell 1 of the present invention is suppressed in the expression of a protein related to endoplasmic reticulum stress. In this case, the expression level of at least one protein related to endoplasmic reticulum stress may be decreased by 10% or more (e.g., 20% or more, 30% or more, 40% or more, 50% or more, 60% or more, 70% or more, 80% or more, 90% or more) compared to the expression level thereof in the cell before suppression.

The protein related to endoplasmic reticulum stress may include, but not limited to, proteins with the following functions:
(1) endoplasmic reticulum stress sensor protein;
(2) protein having the function of promoting formation of disulfide bonds of the target protein;
(3) protein having the function of reducing oxidative stress in cells;
(4) protein having the function of degrading denatured protein;
(5) protein related to endoplasmic reticulum-phagy.

Specific examples of (1) endoplasmic reticulum stress sensor protein include Protein kinase RNA-like ER kinase (PERK, sometimes referred to as "Eif2ak3"). PERK is a type I membrane protein with kinase activity in a cytoplasmic region and is activated by autophosphorylation through dimerization. Activated PERK phosphorylates the translation initiation factor subunit eIF2α. It has been reported that phosphorylation of eIF2α suppresses cap-dependent translation, thus decreasing translation rate and reducing the synthesis of new proteins inserted into the endoplasmic reticulum (B'chir W et al., Nucleic Acids Res. 2013 Sep; 41(16):7683-99.).

Specific examples of (2) protein having the function of promoting formation of disulfide bonds of the target protein (i.e., protein disulfide isomerase) include ER protein 44 (Erp44), Thioredoxin domain containing 12 (Txndc12), and Protein disulfide isomerase family A member 5 (Pdia5). Erp44, which is an endoplasmic reticulum protein, has the function of controlling the oligomer formation and secretion of various proteins and is known to be involved in the quality control of secreted proteins (Hisatsune C et al., Molecular cell, 58(6), 1015-1027). It has been reported that the expression of Txndc12 increases under ER stress induced by unfolded protein response (UPR) (Patricia Inacio et al., EMBO Rep. 2015 Aug; 16(8): 955-964). Pdia5 has been reported to be a protein required when ATF6α responds to ER stress (Arisa Higa et al., Mol Cell Biol. 2014 May; 34(10): 1839-1849).

Specific examples of (3) protein having the function of reducing oxidative stress in cells include Peroxiredoxin-4 (Prdx4). Prdx4 is an antioxidant enzyme belonging to the peroxiredoxin family. It reduces hydrogen peroxide and alkyl hydroperoxides by using the reducing power of glutathione and the like. During antibody formation, it may be used to remove peroxides associated with S-S bond formation. On the other hand, Prdx4 under stress conditions is known to, like PERK, contribute to the rearrangement of the disulfide bonds of ATF6α, an endoplasmic reticulum stress sensor, thereby promoting the efflux of ATF6α from the ER and activating target genes (Arisa Higa et al. (supra)). Furthermore, Prdx4 has been reported to be involved in oxidative stress, ER stress, and protein folding (Evan A Elko et al., J Biol Chem. 2021 Jan-Jun; 296: 100665, and Zito E et al., Mol Cell. 2010 Dec 10; 40(5): 787-797.).

Specific examples of (4) protein having the function of degrading denatured protein include ER degradation enhancing alpha-mannosidase like protein 3 (Edem3) and ER lectin 1 (Erlec1). Edem3 has alpha1,2-mannosidase activity, and is said to be involved in the pruning of mannose attached to proteins in the endoplasmic reticulum and in the endoplasmic reticulum-associated degradation of structurally abnormal glycoproteins (Hirao K et al., J Biol Chem. 2006 Apr 7; 281(14):9650-8.). Erlec1 is a lectin belonging to the endoplasmic reticulum-associated degradation surveillance system and is involved in the degradation of misfolded glycoproteins within the endoplasmic reticulum (Yoshida Y et al., Biochim Biophys Acta. 2010 Feb; 1800(2):172-80.).

Specific examples of (5) protein related to endoplasmic reticulum-phagy include Prolyl 3-hydroxylase family member 4 (P3h4). P3h4 belongs to the Leprecan (leucine proline-enriched proteoglycans) family, which is localized in the endoplasmic reticulum, and is known to regulate the hydroxylation of collagen lysine by forming a complex with prolyl 3-hydroxylase (Ochs RL et al., Mol Biol Cell. 1996 Jul; 7(7):1015-24.; Gruenwald K et al., J Bone Miner Res. 2014 Mar; 29(3):666-75.). In addition, P3h4 is also known to be involved in selective ER-phagy (Ishii S et al., Mol Biol Cell. 2023 Apr 1; 34(4):ar29.).

The means for regulation of the expression of proteins related to endoplasmic reticulum stress in mammalian cells is not particularly limited as long as the expression level of the protein to be regulated can be increased or decreased compared to the expression level of the protein in the mammalian cell before regulation, and a method known per se may be used. For example, a method for increasing the expression level of proteins related to endoplasmic reticulum stress in mammalian cells includes, but is not limited to, a method including transfecting mammalian cells with an expression vector containing a nucleic acid encoding the protein, thereby causing overexpression of the protein in the mammalian cells. In addition, a method for decreasing the expression level of proteins related to endoplasmic reticulum stress in mammalian cells includes, but is not limited to, a method including suppressing the gene expression of the desired proteins related to endoplasmic reticulum stress by knockdown of the gene. Methods for gene knockdown are known per se, and examples include, but are not limited to, antisense methods and RNAi methods using siRNA, shRNA, microRNA, and the like. The knockdown efficiency of the desired protein is not particularly limited, and may be, for example, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, or about 99%. Methods for evaluating whether the protein expression level has been regulated are also known per se, and include, but are not limited to, Western blotting, ELISA, and the like.

In a preferred embodiment, the mammalian cell 1 of the present invention is characterized in that the expression level of at least one selected from the group consisting of PERK, Erp44, Txndc12, Pdia5, Prdx4, Edem3, Erlec1, and P3h4 is suppressed. More preferably, in the mammalian cell 1 of the present invention, the expression level of at least one selected from the group consisting of Erp44, Edem3, Erlec1, and P3h4 is suppressed, and further preferably, the expression level of at least one selected from the group consisting of Erp44, Edem3, and Erlec1 is suppressed.

In the mammalian cell 1 of the present invention, the mammalian cell means a cell derived from a mammal. Examples of mammalian species from which the mammalian cell of the present invention is derived include, but are not limited to, mice, rats, guinea pigs, hamsters, rabbits, cats, dogs, sheep, pigs, cattle, horses, goats, monkeys, and humans. Preferably, the mammal is a hamster or a human.

In one embodiment of the mammalian cell 1 of the present invention, examples of the mammalian cell include, but are not limited to, Chinese hamster ovary (CHO) cells, monkey cell COS-7, human fetal kidney cells (e.g., HEK293 cells), and the like. In one preferred embodiment, the mammalian cell may be a CHO cell.

In the mammalian cell 1 of the present invention, the target protein may be an endogenous protein or an exogenous protein. In one preferred embodiment, the target protein may be an exogenous protein. The exogeneous target protein is not particularly limited, and a high value-added protein is preferred. For example, such proteins include antibodies (natural antibodies, antibody fragments, low molecular weight antibodies, chimeric antibodies, humanized antibodies, human antibodies, and the like), physiologically active proteins (granulocyte colony-stimulating factor (GCSF), granulocyte-macrophage colony-stimulating factor (GM-CSF), erythropoietin, interferon, interleukins such as IL-1 and IL-6, t-PA, urokinase, serum albumin, blood coagulation factors, and the like), cytokines, and the like. In one particularly preferred embodiment, the target protein may be an exogenous protein and may be an antibody or an antigen-binding fragment thereof.

Methods for expressing exogenous proteins in mammalian cells are well known to those of ordinary skill in the art. As one example, the target protein can be produced in mammalian cells by transfecting an expression vector containing a nucleic acid encoding the target protein into mammalian cells and culturing the obtained mammalian cells.

The culture conditions for producing the target protein in mammalian cells vary depending on the type of cells used, and therefore, appropriate conditions can be determined as appropriate. For example, CHO cells can generally be cultured in an atmosphere with a gas phase CO₂ concentration of 0% to 40%, preferably 2% to 10%, the culture medium pH set to pH 6.5 to 7.2, preferably pH 6.7 to 7.0, more preferably pH 6.8 to 6.9, at 30°C to 39°C, preferably around 37°C, for 1 to 50 days, more preferably 1 to 14 days.

The medium for culturing mammalian cells is not particularly limited as long as it allows mammalian cells to survive and produce the target protein. Examples include, but are not limited to, Dulbecco's Modified Eagle's medium (DMEM), Ham's Nutrient Mixture F12, DMEM/F12 medium, McCoy's 5A medium, Eagle's Minimum Essential medium (EMEM), alpha Modified Eagle's Minimum Essential medium (αMEM), MEM medium (Minimum Essential medium), RPMI1640 medium, Iscove's Modified Dulbecco's medium (IMDM), MCDB131 medium, William's Medium E, IPL41 medium, Fischer's medium, E-RDF medium, UltraCHO (registered trademark) medium, EX-CELL (registered trademark) 302 medium, EX-CELL (registered trademark) 325-PF medium, EX-CELL (registered trademark) ACF CHO medium, EX-CELL (registered trademark) CHO DHFR medium, EX-CELL (registered trademark) CD CHO medium, EX-CELL (registered trademark) CD CHO-2 medium, EX-CELL (registered trademark) CD CHO-3 medium, CD OptiCHO (registered trademark) medium, CD CHO medium, CD CHO AGT (registered trademark) medium, CD FortiCHO (registered trademark) medium, CHO-S-SFMII medium, CD DG44 medium, HyClone CDM4CHO medium, HyClone SFM4CHO medium, CD-ACP-CHO, HyClone SFM4CHO-Utillity medium, KBM270 medium, KBM240 medium, KBM230 medium, KBM306 medium, BD Select CHO medium, CHOMACS CD medium, ESF SFM medium, Daigo T medium, IS CHO-V medium (registered trademark), IS CHO-V-GS medium (registered trademark), Opti-Pro, and the like.

A person skilled in the art can add one or more types of various amino acids, various vitamins, energy sources, osmotic pressure regulators, iron sources, pH buffers, antibiotics, and the like in combination to the above-mentioned media. Furthermore, depending on the purpose, a person skilled in the art can also add one or more other chemical or biological components in combination.

As various amino acids, L-alanine, L-arginine, L-asparagine, L-aspartic acid, L-cysteine, L-cystine, L-glutamine, L-glutamic acid, glycine, L-histidine, L-isoleucine, L-leucine, L-lysine, L-methionine, L-ornithine, L-phenylalanine, L-proline, L-serine, L-threonine, L-tryptophan, L-tyrosine, L-valine, and the like can be used. As various vitamins, biotin, folic acid, lipoic acid, nicotinamide, nicotinic acid, p-aminobenzoic acid, calcium pantothenate, pyridoxal hydrochloride, pyridoxine hydrochloride, riboflavin, thiamine hydrochloride, vitamin B12, ascorbic acid, and the like can be used. As energy sources, glucose, galactose, mannose, fructose, and the like can be used. As osmotic pressure regulators, sodium chloride, potassium chloride, potassium nitrate, and the like can be used. As iron sources, EDTA iron, ferric citrate, ferrous chloride, ferric chloride, ferrous sulfate, ferric sulfate, ferric nitrate, and the like can be used. As pH buffers, sodium bicarbonate, calcium chloride, sodium dihydrogen phosphate, HEPES, MOPS, and the like can be used. The addition concentration may be adjusted appropriately according to the type of cells to be cultured and the like.

Examples of antibiotics to be added to the medium include, but are not limited to, sulfonamides, penicillin, pheneticillin, methicillin, oxacillin, cloxacillin, dicloxacillin, flucloxacillin, nafcillin, ampicillin, penicillin, amoxicillin, cyclacillin, carbenicillin, ticarcillin, piperacillin, azlocillin, mexdulocillin, mecillinam, andinocillin, cephalosporins and a derivative thereof, oxolinic acid, amifloxacin, temafloxacin, nalidixic acid, pyromidic acid, ciprofloxacin, cinoxacin, norfloxacin, perfloxacin, rosoxacin, ofloxacin, enoxacin, pipemidic acid, sulbactam, clavulanic acid, β-bromopenicillanic acid, β-chloropenicillanic acid, 6-acetylmethylene-penicillanic acid, cefoxazole, sultanpicillin, adinocillin, tazobactam, aztreonam, sulfazetine, isosulfazetine, norcadisine, m-carboxyphenyl, phenylacetamidophosphonate methyl, chlortetracycline, oxytetracycline, tetracycline, demeclocycline, doxycycline, metacycline, and minocycline. The addition concentration may be adjusted appropriately according to the type of cells to be cultured and the like.

Furthermore, various culture devices for mammalian cell culture can be used, and examples include, but are not limited to, fermentation tank type culture devices, air-lift type culture devices, culture flask type culture devices, spinner flask type culture devices, microcarrier type culture devices, fluidized bed type culture devices, hollow fiber type culture devices, roller bottle type culture devices, packed tank type culture devices, bioreactor type culture devices, and the like.

### 1. Mammalian cell 2 that produces target protein

The present invention provides a mammalian cell that produces a target protein, wherein the expression of a protein related to transcription is regulated in the aforementioned cell (hereinafter sometimes referred to as "the mammalian cell 2 of the present invention").

While the aforementioned mammalian cell 1 of the present invention is characterized in that the expression of a protein related to endoplasmic reticulum stress is regulated, the mammalian cell 2 of the present invention is characterized in that the expression of a protein related to transcription is regulated. In other words, the only difference between the mammalian cell 1 of the present invention and the mammalian cell 2 of the present invention is the protein whose expression is regulated. Therefore, the type of mammalian cell, the type of target protein, the method for regulating protein expression, the culture conditions for mammalian cells, and the like in the mammalian cell 2 of the present invention may be the same as those described for the mammalian cell 1 of the present invention, and definitions and the like can be used by replacing "ER stress" with "transcription."

In the mammalian cell 2 of the present invention, the expression of proteins related to transcription is regulated. The proteins related to transcription are not particularly limited as long as they are proteins that can affect the amount of gene transcription. Examples of such proteins include proteins that constitute basic transcription factors such as TFIID. TFIID is composed of a TATA-binding protein (TBP) and plural types of subunits called TBP-associated factors (TAF).

In one preferred embodiment of the mammalian cell 2 of the present invention, the protein related to transcription may be TAF. Examples of TAF include, but are not limited to, TAF1 (TAFII250), TAF2 (CIF150), TAF3 (TAFII140), TAF4 (TAFII130/135), TAF4B (TAFII105), TAF5 (TAFII100), TAF6 (TAFII70/80), TAF7 (TAFII55), TAF8 (TAFII43), TAF9 (TAFII31/32), TAF9B (TAFII31L), TAF10 (TAFII30), TAF11 (TAFII28), TAF12 (TAFII20/15), TAF13 (TAFII18), TAF15 (TAFII68), and the like. In one preferred embodiment, the protein related to transcription may be TAF8.

In a preferred embodiment, the mammalian cell 2 of the present invention shows enhanced expression levels of a protein related to transcription. In this case, the expression level of the protein related to transcription may be increased by 1.1-fold or more (e.g., 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 3.0-fold or more) compared to the expression level thereof in the cell before enhancement.

In a more preferred embodiment, the mammalian cell 2 of the present invention is characterized by enhanced expression levels of TAF8.

Up until now, the mammalian cells 1 and 2 of the present invention have been described separately based on the function of the protein whose expression level is intended to be regulated (i.e., proteins related to endoplasmic reticulum stress or proteins related to transcription). However, in one embodiment of the present invention, embodiments in which the expression levels of both proteins related to endoplasmic reticulum stress and proteins related to transcription in mammalian cells are regulated simultaneously may also be included in the embodiments of the present invention as long as the desired effects of the present invention are obtained. In one preferred embodiment, the mammalian cell of the present invention is characterized in that the expression level of at least one selected from the group consisting of PERK, Erp44, Txndc12, Pdia5, Prdx4, Edem3, Erlec1, and P3h4 is suppressed, and/or the expression level of TAF8 is enhanced.

Furthermore, mammalian cells that produce a high amount of the target protein can be easily selected by utilizing the characteristics of the mammalian cell of the present invention described above. That is, the present invention also provides a method for selecting mammalian cells that produce a high amount of the target protein (hereinafter sometimes referred to as "the method of the present invention").

The method of the present invention includes quantifying the expression level of at least one selected from the group consisting of PERK, Erp44, Txndc12, Pdia5, Prdx4, Edem3, Erlec1, P3h4 and TAF8 in mammalian cells. The method for quantifying the protein can be a method known per se.

The lower the expression level of at least one selected from the group consisting of PERK, Erp44, Txndc12, Pdia5, Prdx4, Edem3, Erlec1 and P3h4, the mammalian cell can be determined to have a higher production volume of the target protein. Similarly, the higher the expression level of TAF8, the mammalian cell can be determined to have a higher production volume of the target protein.

Furthermore, the target protein can be produced efficiently by utilizing the mammalian cells of the present invention described above. In this respect, the present invention also provides a method for producing a target protein (hereinafter sometimes referred to as "the production method of the present invention").

The production method of the present invention is characterized by culturing the mammalian cell 1 of the present invention and/or the mammalian cell 2 of the present invention under conditions where these cells produce the target protein. Culture conditions preferable for culturing the mammalian cell 1 of the present invention and the mammalian cell 2 of the present invention to produce the target protein may be those described for the mammalian cell 1 of the present invention, but are not limited to those as long as the target protein is produced.

In another embodiment, the present invention provides a target protein produced by the mammalian cell 1 and the mammalian cell 2 of the present invention (hereinafter sometimes referred to as "the target protein of the present invention"). While not wishing to be bound by theory, the mammalian cell 1 of the present invention and the mammalian cell 2 of the present invention are considered to have an intracellular environment favorable for the production of the target protein. Therefore, the target protein produced in such a favorable intracellular environment may also have a high quality.

The present invention is described more specifically in the following examples, but the present invention is not limited in any way by these examples.

### [Example]

### [Example 1]

### Experimental materials and methods

### [Preparation and culture of cell clones]

CHO-DG44 cells adapted to serum-free suspension culture (Urlaub G, et al. Cell. 1983 Jun; 33(2):405-12.) were transfected with vectors containing the genes of two types of human monoclonal antibodies (named IgG1A and IgG1B) (Shibafuji Y, et al. Biotechnol J. 2023 Jul; 18(7):e2200638.), and cloning was performed. From the obtained clones, a total of 12 clones were selected as test subjects: three clones with high productivity and three clones with low productivity expressing IgG1A, and three clones with high productivity and three clones with low productivity expressing IgG1B. Cell passage was performed by adding basal medium warmed to 37°C in a water bath to a 125 mL Erlenmeyer flask and conditioning the medium in a shaker incubator. The cryopreserved vials containing cells were thawed in a hot-water bath, placed in sterile centrifuge tubes containing basal medium, and centrifuged (220 g x 5 min). The supernatant was discarded, and the cell pellet was suspended in a flask containing basal medium to start cell culture. The conditions for the shaker incubator were 37.0°C, 125 rpm (orbital shake diameter 25 mm), and 5% CO₂. Three days after the start of culture, the number of viable cells (VCD) and viability were measured using a Vi-cell BLU (Beckman Coulter). Fed-batch culture of CHO cells: The culture conditions were 37.0°C, 125 rpm (orbital shake diameter 25 mm), and 5% CO₂. Using chemically defined basal medium, each clone (N=3) was cultured in a starting volume of 30 mL. The initial cell density at the start of the fed-batch culture was 0.5x10⁶ cells/mL. Chemically defined feed medium was added from day 3 of culture, and a fixed amount of fed-batch medium was added every three days (Day 3, Day 6, Day 9, Day 12). Harvesting was performed on day 14 of culture. Sampling was performed on Day 3, Day 6, Day 9, Day 12, and Day 14, and culture trend data, titer data, metabolite data, pH, and osmotic pressure were measured. VCD and viability were measured using Vi-cell BLU (Beckman Coulter). For titer analysis and metabolite measurement, the culture sample was centrifuged (9390 g x 1 min) to remove cells, and the analysis was performed using a Cedex Bio HT Analyzer (Roche). pH and osmotic pressure were measured using a pH meter S2K333 (Toyorika) and an automated osmometer OM-6060 (Arkray), respectively.

### [Sample preparation for proteomics analysis]

Sampling was performed from CHO cells in fed-batch culture on Day 6. After centrifugation (14000 g × 30 sec), the supernatant was removed. To wash the cell pellet, washing was performed using 2 mL of ice-cold phosphate-buffered saline (PBS), followed by centrifugation again (14000 g × 30 sec) to remove the PBS. The obtained cell pellet was immediately stored at -80°C.

### [Protein extraction and digestion]

This was performed by modifying a previously reported method (Kawashima Y, et al., J Proteome Res. 2022 Jun 3; 21(6):1418-1427.). Cell pellets were mixed with 2% SDS-100 mM Tris-HCl (pH 8.5), sonicated using a BioRuptor (CosmoBio), and protein concentrations were measured by BCA assay. Twenty micrograms of protein was mixed with tris(2-carboxyethyl)phosphine hydrochloride (TCEP) at a final concentration of 23.8 mM and incubated at 80°C for 10 min. Subsequently, iodoacetamide (IAA) was added at a final concentration of 31.3 mM and alkylated by incubation in the dark at room temperature for 30 min. To this reaction solution, 20 µL of 13.3 µg-solid/µL Sera-mag SpeedBead Carboxylate-Modified Magnetic Particles (a 1:1 mixture of hydrophilic and hydrophobic particles, Cytiva), washed with water, was added and mixed. Then, 99.5% ethanol was added to a final concentration of 71%, and the mixture was mixed for 10 min. The beads were separated on a magnetic stand, the supernatant was discarded, and the beads were washed with 80% ethanol and then with acetonitrile. The air-dried beads were suspended in 100 µL of 50 mM Tris-HCl (pH 8.0) and sonicated for 5 min. Two micrograms of trypsin/LysC mixture (Promega) was added, and the mixture was incubated at 37°C for 20 hr. Quenching was performed by adding 20 µL of 5% TFA and mixing, and the beads were separated using a magnetic stand. The supernatant was collected, desalted using GL-Tip SDB (GL Science), concentrated using SpeedVac (Thermo Fisher Scientific), and then resuspended in 2% acetonitrile-0.1% TFA. The peptide concentration was measured by the absorbance method.

### [LC-MS measurement for proteomics analysis]

For the measurements, a Thermo Scientific EASY-nLC1200 (Thermo Fisher Scientific) and an Orbitrap Eclipse Tribrid mass spectrometer (Thermo Fisher Scientific) were used. As mobile phase A, 0.1% formic acid was used and, as mobile phase B, 0.1% formic acid-80% acetonitrile was used. An Acclam PepMap 100 C18 (75 µm × 12 cm, 3 µm, Thermo Fisher Scientific) was used as the trap column. The sample load was 500 ng, and separation was performed using a NANO HPLC capillary column (75 µm × 12 cm, Nikkyo Technos) at a column temperature of 35°C and a flow rate of 200 nL/min. The gradient conditions were as follows: 6% (0-1 min) - 47% (91 min) - 75% (96 min) - 90% (100-115 min). The ion source spray was set to positive mode and voltage of 2.0 kV. Mass spectra were acquired in overlap mode, DIA mode. The MS1 scan range was set to 495-745 m/z, resolution to 15,000, and target to 4×10⁵. The MS2 spectrum was set to a precursor range of 500-740 or 502-738 m/z, scan range to 200-1800 m/z, resolution to 60,000, target to 1.6×10⁶, and separation window width to 4 m/z.

### [Data analysis for proteomics analysis]

The data acquired by LC-MS was analyzed using DIA-NN version 1.8.1 (Demichev V et al., Nat Methods. 2020 Jan; 17(1):41-44.). First, the measurement result file (raw file) was converted to an mzML file using MSconvert version 3.0.20293 (Kessner D et al., Bioinformatics. 2008 Nov 1; 24(21):2534-6.), and then converted to a dia file using DIA-NN. The spectral library was created from amino acid sequences obtained from Ensembl (Cricetulus griseus CHOK1GS, Ensemble release 104). The DIA-NN analysis parameters were set to a charge range of 2-4, peptide length range of 7-45, precursor ion m/z range of 495-745, fragment ion m/z range of 200-1800, and MS1 and MS2 precision of 10 ppm.

### [Experimental results]

By comparing high-productivity clones and low-productivity clones using proteomics analysis, factors involved in antibody production were comprehensively analyzed. Differences in expression levels were detected for 287 protein factors between high-productivity clone and low-productivity clone. These were categorized into 83 processes based on intracellular biological processes. Furthermore, 10 statistically probable intracellular processes were noted. Categories related to cell proliferation, glycoprotein metabolism, and protein folding were found.

Proteins with expression levels that differ markedly between low- and high-productivity clones are shown in the following Table 1-1 (measured values), Table 1-2 (standardized measured scores for heatmap creation), and Fig. 1 (heatmap).

**[Table 1-1]**

| mgi_symbol | IgGIA-HP1 | IgG1A-HP2 | IgG1A-HP3 | IgG1 B-HP1 | IgG1B-HP2 | IgG1B-HP3 | IgG1A-LP1 | IgG1A-LP2 | IgG1A-LP3 | IgG1B-LP1 | IgG1B-LP2 | IgG1B-LP3 |
|---|---|---|---|---|---|---|---|---|---|---|---|---|
| Prdx4 | 93477200 | 107616000 | 60275700 | 69139400 | 48695700 | 66881600 | 102913000 | 117317000 | 103560000 | 98841500 | 108566000 | 81658100 |
| Txndc12 | 14915000 | 16857500 | 12060200 | 13435400 | 10960600 | 10225800 | 15356000 | 17596100 | 16251800 | 15682300 | 16138300 | 16886500 |
| Pdia5 | 9674230 | 7669910 | 8652510 | 9243820 | 7824130 | 6444240 | 9358120 | 9809320 | 10378800 | 7912270 | 10162500 | 11116200 |
| P3h4 | 3167640 | 3310790 | 3773520 | 3360650 | 2856990 | 2425680 | 5580470 | 3435920 | 4386760 | 3941440 | 4119780 | 3936030 |
| Erp44 | 3486760 | 3520520 | 2821760 | 3266920 | 3110420 | 2997920 | 3502850 | 4174580 | 3873530 | 4301960 | 4188690 | 3921050 |
| Edem3 | 2616340 | 2735000 | 2177370 | 2318240 | 1698140 | 1844660 | 2763560 | 2793110 | 2870740 | 2969100 | 2320310 | 2405890 |
| Erlec1 | 1283210 | 1117760 | 1231000 | 1596350 | 1169830 | 1331060 | 1324380 | 1807800 | 1504110 | 1477660 | 1496530 | 1445730 |
| Eif2ak3 | 488500 | 493354 | 401957 | 372992 | 436773 | 368705 | 496805 | 573404 | 566410 | 491040 | 432563 | 557542 |
| Taf8 | 320927 | 313738 | 395559 | 350871 | 260473 | 286566 | 248170 | 267973 | 250052 | 262014 | 303016 | 280031 |

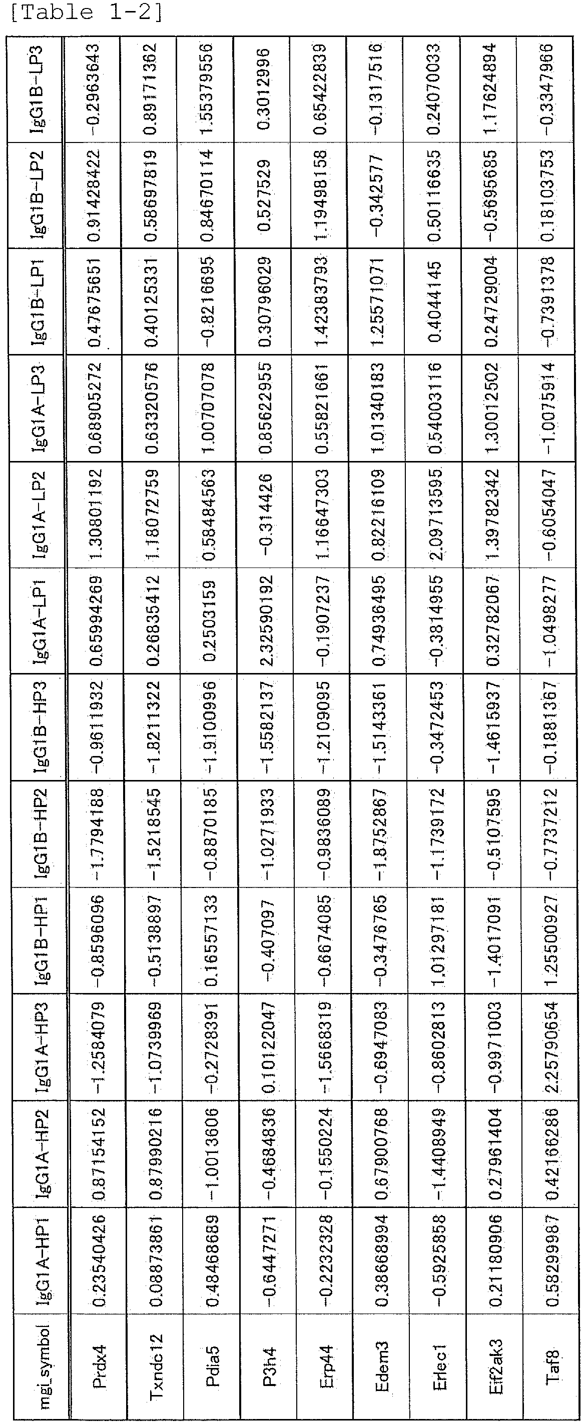

As shown in Table 1-1, Table 1-2, and Fig. 1, the expression levels of the following proteins (1) to (8) were increased in low-productivity clone compared to high-productivity clone.
(1) Prdx4 (Peroxiredoxin-4)
(2) Txndc12 (thioredoxin domain containing 12)
(3) Pdia5 (protein disulfide isomerase family A member 5)
(4) P3h4 (prolyl 3-hydroxylase family member 4)
(5) Erp44 (ER protein 44)
(6) Edem3 (ER degradation enhancing alpha-mannosidase like protein 3)
(7) Erlec1 (ER lectin 1)
(8) PERK (PKR-like ER kinase, also indicated as "Eif2ak3")
   Furthermore, in low-productivity clone, the expression level of protein (9) below was decreased compared to high-productivity clone.
(9) TAF8 (TATA-box binding protein associated factor 8)

(1) to (8) are all proteins related to endoplasmic reticulum stress. (9) is a protein related to transcription.

While not wishing to be bound by theory, the reason for the increased expression level of protein related to endoplasmic reticulum stress in low-productivity clone is considered to be at least partially due to the increased expression level of PERK, one of the sensor proteins important for the endoplasmic reticulum stress response. It is considered that activation of PERK increased the expression levels of protein related to endoplasmic reticulum stress and located downstream of its signal cascade, such as Prdx4, Erp44, Txndc12, Pdia5, P3h4, Edem3, and Erlec1.

### [Example 2]

### Experimental materials and methods

### [Preparation of gene knockdown pool using shRNA]

Of the target genes selected by proteomics analysis, eight genes excluding PERK (TAF8, Pdia5, P3h4, Edem3, Prdx4, Txndc12, Erp44, and Erlec1) were subjected to gene knockdown using shRNA. ShRNA expression vector targeting sequence not present in the CHO cell genome sequence was used as the control. Preparation of these shRNA expression vectors was committed to VectorBuilder Inc. (Chicago, IL, USA). An overview of each expression vector prepared is shown in Table 2 and Table 3 below. As one example, Fig. 2 shows the vector map of the shRNA expression vector targeting the TAF8 gene. The nucleic acid sequences of shRNA expression vector targeting the TAF8 gene and shRNA expression vector targeting genes other than TAF8 are identical except for the shRNA nucleic acid sequence. Detailed information of each shRNA expression vector can be obtained from vectorbuilder.com using the vector IDs shown in Table 2 and Table 3. The day before transfection, the IgG1A and IgG1B expression clones constructed above were subcultured in chemically defined basal medium to VCD of 0.5×10⁶ cells/mL. After 1 day of culture, the culture medium was divided into 10×10⁶ cells and centrifuged at 220 × g for 5 min. The supernatant was discarded and the cell pellet was collected. The cells were washed with D-PBS, and then centrifuged again at 220 × g for 5 min. The supernatant was discarded and the cell pellet was collected. The cell pellet was resuspended in 100 µL of Neon Transfection Buffer R (Thermofisher Scientific), mixed with 10 µg of shRNA expression vector, and transfection was performed by electroporation using Neon Transfection System (Thermofisher Scientific) to create a knockdown pool for each gene.

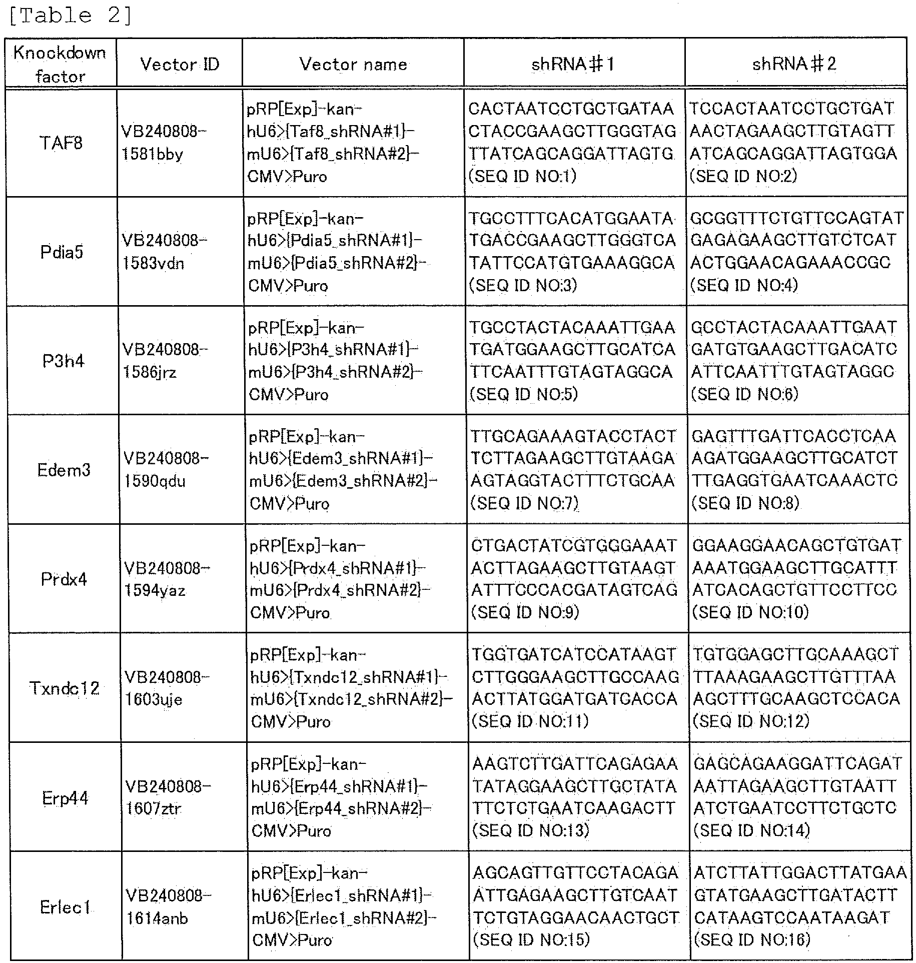

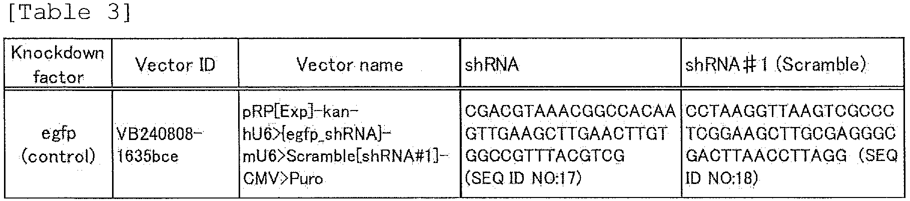

### [Batch culture of knockdown CHO cell pool]

The culture conditions were 37.0°C, 125 rpm (orbital shake diameter 25 mm), and 5% CO₂. Using chemically defined basal medium, each pool was cultured in a starting volume of 10 mL after electroporation. Samples were taken daily from the day of transfection (Day 0) to Day 4, and VCD, Viability, and titer data were measured. VCD and Viability were measured using Vi-cell BLU (Beckman Coulter). Titer analysis was performed using Octet (SARTORIUS).

### [Analysis of mRNA amount]

mRNA extraction was performed on the cultured cells subjected to the above-mentioned batch culture, according to the manufacturer's protocol, using RNeasy Mini Kit (QIAGEN). A portion of the obtained RNA solution was taken, and the concentration was measured by absorbance using NanoDrop (Thermofisher Scientific). Furthermore, the degree of RNA degradation was measured using TapeStation 4200 (Agilent). RNA concentrations were equalized between samples, and cDNA was synthesized using iScript AdV cDNA Kit for RT-qPCR (Bio-Rad) according to the manufacturer's protocol. Using the prepared cDNA as a template, real-time PCR was performed using THUNDERBIRD SYBR qPCR Mix (TOYOBO). Real-time PCR was performed using the CFX96 Touch real-time PCR analysis system (Bio-Rad). The amount of Chinese Hamster Cyclophyllin (Cyc) mRNA was measured for each sample, and the measurement results for TAF8, Pdia5, P3h4, Edem3, Prdx4, Txndc12, Erp44, and Erlec1 were normalized.

### [Experimental results]

For the above-mentioned target genes selected by proteomics analysis, knockdown pools were prepared and they were subjected to culture evaluation. As a result, the antibody production level increased in the pools in which Pdia5, P3h4, Edem3, Prdx4, Txndc12, Erp44, and Erlec1 were knocked down compared to the control. In addition, the antibody production level decreased in the pool in which TAF8 was knocked down compared to the control (Fig. 3, Fig. 4, Fig. 5, Fig. 6). The ratio of the mRNA level of the relevant factor in each knockdown pool, where the mRNA level of the relevant factor in the control group is set to 1, is shown in Table 4. As shown in Table 4, in all the cells into which shRNA vectors for TAF8, Pdia5, P3h4, Edem3, Prdx4, Txndc12, Erp44, and Erlec1 were introduced, suppression of the expression level of the relevant factor was observed compared to the control group. From these results, it was found that suppressing gene expression for Pdia5, P3h4, Edem3, Prdx4, Txndc12, Erp44, and Erlec1 improves the antibody production level. Furthermore, in the case of TAF8, suppressing gene expression level decreased the antibody production level, suggesting the possibility of improving the antibody production level by enhancing its gene expression.

**[Table 4]**

| Factor | Ratio |
|---|---|
| Taf8 | 0.40 |
| Pdia5 | 0.75 |
| P3h4 | 0.07 |
| Edem3 | 0.51 |
| Prdx4 | 0.13 |
| Txndc12 | 0.06 |
| Erp44 | 0.04 |
| Erlec1 | 0.16 |

The results of the examples are considered to succinctly describe the characteristics of cell lines having low production levels of the target protein. The results of the examples show that cells having a high production level of the target protein can be easily and accurately selected by using the expression levels of proteins related to endoplasmic reticulum stress and proteins related to transcription as indicators. In addition, mammalian cells having high production levels of the target protein can be easily produced by artificially reducing the expression levels of the aforementioned proteins related to endoplasmic reticulum stress or artificially increasing the expression level of the aforementioned protein related to transcription.

### [Industrial Applicability]

According to the present invention, cells that produce a high amount of the target protein can be easily selected or produced. Therefore, the present invention is extremely useful in the fields of medicine, pharmacy, protein science, and the like.

This application is based on a patent application No. 2023-211379 filed in Japan (filing date: December 14, 2023), the contents of which are incorporated in full herein.

## Claims

1. A mammalian cell that produces a target protein, wherein the expression of a protein related to endoplasmic reticulum stress is regulated in the aforementioned cell.

2. The cell according to claim 1, wherein the aforementioned mammalian cell is a Chinese hamster ovary (CHO) cell.

3. The cell according to claim 1 or 2, wherein the aforementioned target protein is an antibody.

4. The cell according to claim 1 or 2, wherein the expression of the aforementioned protein related to the endoplasmic reticulum stress is suppressed.

5. The cell according to claim 1 or 2, wherein the aforementioned protein related to the endoplasmic reticulum stress is an endoplasmic reticulum stress sensor protein.

6. The cell according to claim 5, wherein the endoplasmic reticulum stress sensor protein is PERK.

7. The cell according to claim 1 or 2, wherein the aforementioned protein related to the endoplasmic reticulum stress is a protein that has the function of promoting the formation of a disulfide bond of the target protein.

8. The cell according to claim 7, wherein the aforementioned protein that has the function of promoting the formation of a disulfide bond of the target protein is at least one selected from the group consisting of Erp44, Txndc12, and Pdia5.

9. The cell according to claim 1 or 2, wherein the aforementioned protein related to the endoplasmic reticulum stress is a protein that has the function of reducing oxidative stress.

10. The cell according to claim 9, wherein the aforementioned protein that has the function of reducing oxidative stress is Prdx4.

11. The cell according to claim 1 or 2, wherein the aforementioned protein related to the endoplasmic reticulum stress is a protein that has the function of degrading a denatured protein.

12. The cell according to claim 11, wherein the aforementioned protein that has the function of degrading a denatured protein is at least one selected from the group consisting of Edem3 and Erlec1.

13. The cell according to claim 1 or 2, wherein the aforementioned protein related to the endoplasmic reticulum stress is a protein related to endoplasmic reticulum-phagy.

14. The cell according to claim 13, wherein the aforementioned protein related to the endoplasmic reticulum-phagy is P3h4.

15. A mammalian cell that produces a target protein, wherein the expression of a protein related to transcription is regulated in the aforementioned cell.

16. The cell according to claim 15, wherein the aforementioned mammalian cell is a Chinese hamster ovary (CHO) cell.

17. The cell according to claim 15 or 16, wherein the aforementioned target protein is an antibody.

18. The cell according to claim 15 or 16, wherein the expression of the aforementioned protein related to the transcription is enhanced.

19. The cell according to claim 15 or 16, wherein the aforementioned protein related to the transcription is TAF8.

20. A method for producing a target protein, comprising a step of culturing the cell according to claim 1 or 15.

21. A target protein produced by the cell according to claim 1 or 15.
